**Europäisches Patentamt**

**European Patent Office**

**Office européen des brevets**

(19)

(11) Veröffentlichungsnummer: **0 241 760**
**B1**

(12)

# EUROPÄISCHE PATENTSCHRIFT

(45) Veröffentlichungstag der Patentschrift:
**19.12.90**

(21) Anmeldenummer: **87104204.0**

(22) Anmeldetag: **21.03.87**

(51) Int. Cl.⁵: **B01J 23/60,** C07C 29/136

(54) **Katalysator und Verfahren zur katalytischen Hydrierung von Fettsäuremethylestern im Festbett.**

(30) Priorität: **29.03.86  DE 3610698**

(43) Veröffentlichungstag der Anmeldung:
**21.10.87 Patentblatt 87/43**

(45) Bekanntmachung des Hinweises auf die Patenterteilung:
**19.12.90 Patentblatt 90/51**

(84) Benannte Vertragsstaaten:
**BE DE ES FR GB IT NL SE**

(56) Entgegenhaltungen:
**EP-A- 0 055 512**
**EP-A- 0 085 398**
**DE-A- 2 613 226**
**FR-A- 1 392 376**
**US-A- 3 524 899**
**US-A- 4 113 662**
**US-A- 4 210 561**

(73) Patentinhaber: **Henkel Kommanditgesellschaft auf Aktien, Postfach 1100 Henkelstrasse 67, D-4000 Düsseldorf-Holthausen(DE)**

(72) Erfinder: **Schütt, Hartwig, Dr., Haydnstrasse 50, D-4000 Düsseldorf-Benrath(DE)**
Erfinder: **Reinhold, Karl, Dieselstrasse 43, D-5650 Solingen 19(DE)**

## Beschreibung

Die Erfindung betrifft ein Verfahren zur katalytischen Hydrierung von Fettsäuremethylestern im Festbett und einen für ein derartiges Verfahren geeigneten Katalysator.

Fettalkohole, d.h. überwiegend lineare, monofunktionelle, endständige Alkohole mit Alkylresten einer Kettenlänge von 8 bis 18 C-Atomen sowie Verfahren zu ihrer Herstellung sind literaturbekannt. Das Herstellungsverfahren wird beispielsweise in "Ullmanns Enzyklopädie der technischen Chemie", 4. Auflage, Band 11, Seiten 427 ff, beschrieben. Ein bevorzugtes Ausgangsmaterial zur Gewinnung derartiger Fettalkohole sind die in Fetten und/oder Ölen natürlichen Ursprungs vorkommenden Fettsäuren bzw. Fettsäuregemische einer Kettenlänge von 8 bis 18 C-Atomen, die als Methylester durch katalytische hydrierende Reaktion in Fettalkohole entsprechender Kettenlänge umgewandelt werden.

In den bekannten Verfahren werden Fettalkohole aus Fettsäuremethylestern nach dem sogenannten "Festbett-Hydrierverfahren" hergestellt, wobei Fettsäuremethylester bei Temperaturen von mindestens 200°C und Drucken von mindestens 25 bar, vorzugsweise bei Temperaturen von 230 bis 250°C und Drucken von 250 bis 300 bar zusammen mit großen Überschußmengen Wasserstoff über feste, zumeist kupferhaltige Mischoxid-Katalysatoren geleitet werden (siehe beispielsweise DE-AS 10 05 497, DE-AS 25 13 377 und DE-AS 26 13 226). Die in diesem Verfahren verwendeten festen Katalysatoren sind in ihrer aktivierten Form luftempfindlich und können deshalb nicht gelagert und transportiert werden. Aus diesem Grund werden die Katalysatoren unmittelbar vor dem Gebrauch in der Anlage zur Hydrierung von Fettsäuremethylestern durch Reduktion mit Wasserstoff aktiviert. Die Katalysatoraktivierung erfordert lange Vorlaufzeiten vor dem eigentlichen Hydrierprozeß und beeinträchtigt die Raum-/Zeitausbeute der Anlagen erheblich.

Es bestand daher ein Bedarf für ein im großtechnischen Maßstab mit Vorteil durchführbares Verfahren sowie geeignete Katalysatoren zur katalytischen reduzierenden Hydrierung von Fettsäurealkylestern zu langkettigen linearen Fettalkoholen, die die Reaktionsprodukte bei hoher Raum-/Zeitausbeute mit großer Selektivität zugänglich machen. Die in derartigen Verfahren verwendeten Katalysatoren sollten preiswert zugänglich sein und zudem nach entsprechender Aufarbeitung bzw. schneller Reaktivierung leicht wiederverwertet werden können. Eine Verkürzung der für die Hydrierung erforderlichen Reaktionszeiten war ebenfalls erwünscht.

Überraschend wurde nun gefunden, daß sich Raum-/Zeitausbeute und Selektivität der katalytischen reduzierenden Festbett-Hydrierung von Fettsäurealkylestern deutlich verbessern lassen, wenn man die als Katalysator verwendeten Zinkoxidstücke mit geringen Mengen von Übergangsmetallen aus der achten Nebengruppe des Periodensystems dotiert und den auf diesem Wege gewonnenen festen Katalysator zur reduzierenden Hydrierung von Fettsäurealkylestern zu linearen gesättigten Fettalkoholen einsetzt.

Die Erfindung betrifft feste, stückige Katalysatoren auf Zinkoxidbasis für die reduzierende katalytische Hydrierung von Fettsäuremethylestern und deren Mischungen zu gesättigten Fettalkoholen und deren Mischungen im Festbett, die dadurch gekennzeichnet sind, daß sie nach Einwirken wässriger Lösungen alkalistabiler Komplexsalze der Übergangsmetalle Ruthenium, Osmium, Rhodium, Iridium, Palladium und/oder Platin auf festes stückiges Zinkoxid und anschließende Reduktion mit gasförmigem Wasserstoff 0,01 bis 1,0 Gew.-% eines oder mehrerer der genannten Übergangsmetalle gleichmäßig in der festen Katalysatorbasis verteilt enthalten.

Die Erfindung betrifft außerdem ein Verfahren zur katalytischen reduzierenden Hydrierung von Fettsäurealkylestern und/oder deren Mischungen zu gesättigten Fettalkoholen und/oder deren Mischungen im Festbett mit Wasserstoff unter Verwendung eines festen, stückigen Katalysators auf Zinkoxidbasis, das dadurch gekennzeichnet ist, daß man festes stückiges Zinkoxid mit einer wässrigen Lösung eines oder mehrerer alkalistabiler Komplexsalze der Übergangsmetalle Ruthenium, Osmium, Rhodium, Iridium, Palladium und/oder Platin tränkt, die so erhaltenen Übergangsmetalloxid-beaufschlagten Zinkoxidstücke nach Trocknung in kurzer Zeit im Wasserstoffstrom bei erhöhter Temperatur reduziert und Fettsäurealkylester und/oder deren Mischungen unter Verwendung des so erhaltenen festen Katalysators bei erhöhter Temperatur und erhöhtem Wasserstoffdruck kontinuierlich oder diskontinuierlich zu gesättigten Fettalkoholen und/oder deren Mischungen hydriert.

Als Basis für die erfindungsgemäßen festen stückigen Katalysatoren wird Zinkoxid verwendet. Dabei sind im Prinzip beliebige Formkörper von Zinkoxid geeignet, beispielsweise Strangpreßlinge, Tabletten, Pellets oder auch durch Ansinterung verfestigte Formkörper.

Bevorzugt wird als Basis für die erfindungsgemäßen Katalysatoren stranggepreßtes Zinkoxid-Extrudat verwendet, dessen Stücke einen Durchmesser von 4,5 mm und ein Schüttgewicht von etwa 1,4 aufweisen. Derartiges Zinkoxid-Extrudat wird beispielsweise auch als Entschwefelungskatalysator eingesetzt. Es ist jedoch auch mit gleichem Erfolg festes Zinkoxid anderer Festkörper-Form für die Katalysatoren gemäß der Erfindung verwendbar.

Zinkoxid-Festkörper der genannten Art werden zur Herstellung eines für die reduzierende Hydrierung von Fettsäuremethylestern zu Fettalkoholen geeigneten Katalysators mit wässrigen Lösungen alkalistabiler Komplexsalze der Übergangsmetalle Ruthenium, Osmium, Rhodium, Iridium, Palladium und/oder Platin in Kontakt gebracht. Es können eine oder mehrere Lösungen alkalistabiler Komplexsalze der genannten Übergangsmetalle verwendet werden. Bevorzugt läßt man alkalistabile Komplexsalze des Palladiums auf die Zinkoxid-Festkörper einwirken.

Der Vorgang des Einwirkens kann auf beliebige Art und Weise erfolgen, beispielsweise durch Tauchen, Besprühen, Tränken usw. Sichergestellt werden muß in jedem Fall, daß der gesamte Zinkoxid-Festkörper gleichmäßig mit der wässrigen Lösung des Übergangsmetallkomplexsalzes durchdrungen wird und sich die genannten Übergangsmetalle nicht in Form ihrer Oxide bereits an der Oberfläche der Formkörper niederschlagen und dadurch eine ungleichmäßige Verteilung der Übergangsmetalle im Zinkoxid-Festkörper erreicht wird. Derartige Katalysatoren würden nur eine unbefriedigende Durchsatzleistung in der Fettsäurealkylester-Hydrierung erbringen, die auch durch eine Temperatursteigerung nicht zufriedenstellend verbessert werden kann. Bevorzugt werden die Zinkoxid-Festkörper über längere Zeit, beispielsweise 1 bis 5 Stunden, bevorzugt 3 Stunden, in geeignete Übergangsmetallsalz-Lösungen eingetaucht, so daß der gesamte Katalysator-Festkörper mit der verdünnten Komplexsalzlösung getränkt ist.

Entscheidend für die Auswahl geeigneter Übergangsmetallsalze ist ihre gute Alkalistabilität. Da Zinkoxid basisch reagiert, würden schlecht alkalistabile Übergangsmetallsalze auf der Oberfläche des Zinkoxidformkörpers in die entsprechenden Übergangsmetalloxide überführt. Damit wäre eine durchdringende Tränkung des Zinkoxid-Festkörpers mit Übergangsmetallsalz nicht gewährleistet. Vielmehr würde das Übergangsmetall bereits auf der Oberfläche des stückigen Zinkoxids als Oxid ausgefällt. Ausreichend alkalistabile Komplexsalze der genannten Übergangsmetalle, die mit Vorteil zur Tränkung des stückigen Zinkoxids verwendet werden können, sind beispielsweise Ammoniumhexachlororuthenat(IV), Ammoniumhexachloroosmat(IV), Ammoniumhexachlororhodat(IV), Tetrammin-palladium(II)-chlorid, Diammindinitropalladium(II), Bis(acetylacetonato)palladium(II), Tetramminpalladium(II)-tetrachloropalladat(II) (Vauquelin'sches Salz), Ammoniumtetrachloroplatinat(II), Ammoniumhexachloroplatinat(IV), cis-Diammindichloroplatin(II), trans-Diammindichloroplatin(II), cis-Diammintetrachloroplatin(IV), Diammindinitritoplatin(II), Hexabromoplatin(IV)-säurenonahydrat, Kaliumhexabromoplatinat(IV), Kaliumhexachloroplatinat(IV), Kaliumhexaiodoplatinat(IV), Kaliumtetrabromoplatinat(II)-dihydrat, Kaliumtetrachloroplatinat(II), Kaliumtetranitritoplatinat(II), Natriumhexachloroplatinat(IV)-hexahydrat, Natriumtetrachloroplatinat(II)-tetrahydrat, Tetraamminplatin(II)-chloridmonohydrat, Tetraamminplatin(II)-nitrat, Kaliumhexacyanoplatinat(IV), Kaliumtetracyanoplatinat(II)-hydrat, Bis(acetylacetonato)platin(II), Dichloro(ethylendiamin)platin(II), Tetrachloro(ethylendiamin)platin(IV).

Vorzugsweise und mit besonderem Erfolg werden aus der oben genannten Gruppe die alkalistabilen Komplexsalze des Palladiums verwendet. Bevorzugte Übergangsmetall-Komplexsalze sind aufgrund ihrer verhältnismäßig einfachen Verfügbarkeit und der mit ihnen erzielbaren guten Hydrierergebnisse Tetramminpalladium(II)-chlorid, Diammindinitropalladium(II), Bis(acetylacetonato)palladium(II) und Tetramminpalladium(II)-tetrachloropalladat(II) (Vauquelin'sches Salz).

Entscheidendes Kriterium für die Auswahl der geeigneten Übergangsmetall-Komplexsalze ist ihre gute Löslichkeit in Wasser oder mit Wasser mischbaren Lösungsmitteln sowie eine ausreichende Alkalistabilität. Nur dann, wenn der gesamte (in Gegenwart wässriger Medien alkalisch reagierende) Zinkoxid-Festkörper vollständig mit Komplexsalzlösung durchtränkt werden kann, ohne daß sich die Übergangsmetalle in Form ihrer Oxide niederschlagen, ist eine genügende Alkalistabilität gegeben. Experimentell läßt sich dies am einfachsten dadurch überprüfen, daß bei dem (unten näher beschriebenen) Vorgang des Tränkens des Zinkoxidfestkörpers nach ausreichender Einwirkzeit von beispielsweise 3 h die Übergangsmetall-Komplexsalz-Lösung abdekantiert und der Festkörper getrocknet und im Wasserstoffstrom reduziert wird. Beim Aufbrechen des reduzierten Festkörpers muß dann die gesamte Bruchfläche gleichmäßig durch ausgeschiedenes Edelmetall grau oder schwarzgrau gefärbt sein.

Für den Vorgang des Tränkens des stückigen Zinkoxids mit Übergangsmetall-Komplexsalz-Lösung wird die Konzentration der Übergangsmetall-Komplexsalze in den wässrigen Lösungen so eingestellt, daß die Menge an Übergangsmetall, bezogen auf die feste Katalysatorbasis, im Bereich von 0,01 bis 1,0 Gew.-% der genannten Übergangsmetalle liegt. Mit anderen Worten: Es werden Übergangsmetallsalz-Lösungen einer solchen Konzentration eingesetzt, daß der für die Hydrierung verwendete Katalysator 0,01 bis 1,0 Gew.-% Übergangsmetall enthält. Bevorzugter Bereich der Übergangsmetallkonzentration im Katalysator ist dabei etwa 0,1 Gew.-% des Edelmetalls im Katalysator. Die anzuwendende Konzentration der Salze in den wässrigen Lösungen ist u.a. von den Eigenschaften des verwendeten ZinkoxidFestkörpers (Oberfläche, Durchmesser, Schüttgewicht) abhängig und kann im Rahmen einfacher Vorversuche schnell ermittelt werden.

Die erfindungsgemäßen Katalysatoren werden dadurch hergestellt, daß man die mit dem Übergangsmetall-Komplexsalz beaufschlagten Zinkoxidstücke nach Abdekantieren der überschüssigen Lösung und Trocknung im Wasserstoffstrom bei erhöhter Temperatur reduziert. Der Trockungsprozeß erfolgt bei einer Temperatur im Bereich von 100 bis 150°C für ungefähr eine Stunde, während die Reduktion im Wasserstoffstrom bei Temperaturen im Bereich von 200 bis 250°C über 10 bis 24 Stunden durchgeführt wird. Bevorzugte Trocknungstemperatur ist 130°C. Die Reduktion wird mit Vorteil bei 210 bis 240°C über 18 bis 20 Stunden durchgeführt. Die erhaltenen festen stückigen Katalysatoren auf Zinkoxidbasis enthalten danach 0,01 bis 1,0 Gew.-% eines oder mehrerer der genannten Übergangsmetalle, bevorzugt 0,1 Gew.-% gleichmäßig in der festen Katalysatorbasis verteilt.

Die so erhaltenen Katalysatoren sind hervorragend zur reduzierenden katalytischen Hydrierung von Fettsäurealkylestern und/oder deren Mischungen zu gesättigten Fettalkoholen und/oder deren Mischungen im Festbett geeignet. Dazu werden Fettstoffe nativer oder synthetischer Herkunft nach an sich bekannten Methoden mit Methanol umgesetzt und dadurch in die entsprechenden Fettsäuremethylester bzw. deren Mischungen überführt. Unter Verwendung der oben beschriebenen festen, stückigen Katalysatoren auf Zinkoxidbasis, die mit geringen Mengen der genannten Übergangsmetalle beaufschlagt sind, werden die Fettsäuremethylester bzw. deren Mischungen der hydrierenden Reduktion in Gegenwart molekularen Wasserstoffs unterworfen. Diese Umsetzung kann kontinuierlich oder diskontinuierlich durchgeführt werden. Erhöhte Temperaturen und/oder erhöhter Wasserstoffdruck beschleunigen die Reaktion, die mit Vorteil bei Temperaturen im Bereich von 200 bis 300°C und Wasserstoffdrücken von 200 bis 300 bar, bevorzugt bei 270 bis 290°C und 250 bar Wasserstoffdruck, durchgeführt wird.

Es entstehen in hohen Ausbeuten die gewünschten linearen gesättigten Fettalkohole, wobei die Raum-/Zeitausbeuten gegenüber den bisherigen Methoden der reduzierenden katalytischen Hydrierung im Festbett ausgesprochen hoch liegen und in Spitzenbereichen - abhängig vom verwendeten Katalysator sowie den jeweiligen Reaktionsbedingungen - Umsetzungsgrade von 95 bis 98 % der Theorie erreicht werden.

Neben der hohen Selektivität der erfindungsgemäßen Katalysatoren bzw. der Möglichkeit, mit ihrer Hilfe in der reduzierenden katalytischen Hydrierung von Fettsäuremethylestern zu linearen gesättigten Fettalkoholen hohe Raum-/Zeitausbeuten zu erreichen, ist es als wesentlicher Vorteil der Katalysatoren anzusehen, daß sie nach Tränkung des stückigen Zinkoxids mit den Komplexsalzen der genannten Übergangsmetalle und Aktivierung mit Wasserstoff eine außerordentlich hohe Lagerstabilität aufweisen. Der durch Umsetzung mit Wasserstoff aktivierte Katalysator ist praktisch unbegrenzt lagerstabil und bedarf einer zusätzlichen Stabilisierung gegen Einflüsse durch Luft und/oder Feuchtigkeit bzw. mechanische Einwirkungen nicht. Dies ergibt sich aus der Tatsache, daß die nach Reduktion mit molekularem Wasserstoff in elementarer Form vorliegenden Edelmetalle der Platin-Gruppe (Gruppe 8b des PSE) durch Luftsauerstoff nicht oxidiert werden. Eine erneute Aktivierung, auch nach längeren Lagerzeiten, ist somit nicht erforderlich.

Als zusätzlicher Vorteil für das vorliegende Hydrierverfahren unter Einsatz der erfindungsgemäßen Katalysatoren ist es anzusehen, daß der Zeit- und Energieaufwand für den Aktivierungsprozeß durch Reduktion mit Wasserstoff wesentlich niedriger ist als für bisher bekannte Festbett-Katalysatoren, wie beispielsweise kupferhaltige Systeme. In Abhängigkeit von Katalysatorbasis (Zinkoxid), Übergangsmetall-Komplexsalz und Reaktionsbedingungen bei der Aktivierung läßt sich der Aufwand auf 1/10 der bisher notwendigen Zeit/Energie reduzieren.

In nicht aktivierter Form sind die erfindungsgemäßen Katalysatoren ebenfalls ausgezeichnet handhabbar und zeigen alle Vorteile, die sie für ein technisches Verfahren geeignet machen.

Ein weiterer Vorteil der erfindungsgemäßen Katalysatoren ist darin zu sehen, daß sie auch gegen im Katalysesystem gegebenenfalls vorliegende Katalysatorgifte ausgesprochen unempfindlich sind. Die Wirksamkeit von Zinkoxid als Entgiftungskatalysator ist allgemein bekannt. Auch in einem in Gegenwart von Katalysatorgiften ablaufenden Hydrierprozeß gemäß der vorliegenden Erfindung wirkt der Zinkoxidanteil im Katalysator insofern entgiftend, als er Katalysatorgifte wirksam bindet. Damit wird eine vorzeitige Alterung bzw. Desaktivierung des Katalysators durch Katalysatorgifte verhindert.

Die erfindungsgemäßen Katalysatoren kommen zudem einem starken Bedürfnis der Anwender entgegen, nach Verbrauch des Katalysators nach längerer Einsatzzeit die teuren Übergangsmetall-Anteile wiederzugewinnen. Dies ist mit den erfindungsgemäßen Katalysatoren auf Zinkoxidbasis insofern leichter als mit bisher gekannten Katalysatoren möglich, als das Katalysatormaterial unter Verwendung von Mineralsäuren leicht aufgelöst und die Zinkanteile in der Lösung leicht von den Übergangsmetall-Anteilen Abgetrennt werden können. Die hochkonzentrierte Anreicherung der Platinmetalle läßt sich dann leicht wiederaufarbeiten und für eine erneute Herstellung erfindungsgemäßer Katalysatoren verwenden.

Die Erfindung wird durch die nachfolgenden Beispiele näher erläutert.

### Beispiel 1

Katalysatorherstellung; Übergangsmetall: Palladium.

0,61 g $PdCl_2$ wurden in 100 ml Wasser unter Zusatz von drei Tropfen konzentrierter Salzsäure kalt gelöst. Danach wurden 3 g konzentrierte Ammoniumhydroxidlösung zugetropft und die Reaktionsmischung auf 80°C erwärmt. Es entstand eine klare Lösung, die mit Wasser auf 120 ml aufgefüllt wurde. Darin wurden dann 180 g Zinkoxidpreßlinge 3 h lang getränkt. Unter Zinkoxid ist in diesem wie auch den folgenden Beispielen stranggepreßtes Zinkoxid-Extrudat (4,5 mm Durchmesser; Schüttgewicht: 1,4) zu verstehen. Ein derartiges Zinkoxid-Extrudat wird auch technisch als Entschwefelungskatalysator eingesetzt.

Nach einer Tränkzeit von 3 h wurden 60 ml der Übergangsmetallsalz-Lösung abdekantiert und das mit Übergangsmetall-Komplexsalz beaufschlagte Zinkoxid bei 130°C getrocknet. Der getrocknete Katalysator hatte einen Palladiumgehalt von 0,1 Gew.-%, bezogen auf Zinkoxid-Katalysatorbasis.

## Beispiel 2

Katalysatorherstellung; Übergangsmetall: Palladium.

1 g Bis(acetylacetonato)palladium(II) wurde in 110 ml Wasser und 10 ml Ammoniumhydroxid heiß gelöst und in dieser Lösung 180 g stückiges Zinkoxid getränkt. Nach 3 h wurden 60 ml überschüssiger Übergangsmetall-Lösung abdekantiert und der mit Palladium-Komplexsalz beaufschlagte Zinkoxidkatalysator bei 130°C getrocknet. Der getrocknete Katalysator hatte einen Palladiumgehalt von 0,1 Gew.-%, bezogen auf Zinkoxid-Katalysatorbasis.

## Beispiel 3

Katalysatorherstellung; Übergangsmetall: Platin.

0,96 g $H_2(PtCl_6)$ . 6 $H_2O$ wurden in 100 ml Wasser gelöst. Dieser Lösung wurde bis zum Erreichen eines pH-Wertes von 10 konzentrierte Ammoniumhydroxidlösung zugetropft. Die resultierende Lösung wurde mit Wasser auf 120 ml aufgefüllt und darin 180 g Zinkoxidpreßlinge 3 h lang getränkt. Nach 3 h wurden 60 ml der überstehenden Lösung abdekantiert und das mit Übergangsmetall-Komplexsalz beaufschlagte Zinkoxid bei 130 °C getrocknet. Der getrocknete Katalysator hatte einen Platingehalt von 0,1 Gew.-%, bezogen auf Zinkoxid-Katalysatorbasis.

## Beispiel 4

Katalysatorherstellung; Übergangsmetall: Ruthenium.

5 g Weinsäure wurden in 10 g Wasser gelöst. Anschließend wurde diese Lösung mit soviel konzentrierter Kalilauge versetzt, bis eine klare Lösung entstanden war. Die resultierende Kaliumtartratlösung hatte einen pH-Wert von 10. Dieser Lösung wurden 0,72 g $RuCl_3$ im Verlauf von mehreren Stunden in der Hitze zugegeben und die resultierende Lösung mit Wasser auf 120 ml aufgefüllt, wobei sich ein pH-Wert von 6 einstellte.

In der resultierenden Lösung von Rutheniumtartratochlorid wurden dann 180 g Zinkoxidpreßlinge 3 h lang aufbewahrt, so daß sie vollständig mit der Übergangsmetall-Komplexsalz-Lösung getränkt waren. Danach wurden 60 ml der Übergangsmetall-Komplexsalz-Lösung abdekantiert und das mit Übergangsmetall-Komplexverbindung beaufschlagte Zinkoxid bei 130°C getrocknet. Die Konzentration an Ruthenium in der Zinkoxid-Katalysatorbasis betrug 0,1 Gew.-%.

## Beispiel 5

Katalysatorherstellung; Übergangsmetall: Osmium.

5 g Weinsäure wurden in 10 ml Wasser gelöst und dann mit soviel konzentrierter Kalilauge versetzt, bis die Lösung klar war und sich ein pH-Wert von 10 eingestellt hatte. Außerdem wurden 0,56 g $OsCl_3$ in 50 ml Wasser gelöst. Beide wässrigen Lösungen wurden vereinigt, auf 120 ml mit Wasser aufgefüllt und 1 h lang in der Hitze gerührt. In der resultierenden Komplexsalzlösung wurden dann 180 g Zinkoxidpreßlinge 3 h lang aufbewahrt, bis sie vollständig getränkt waren. Danach wurden 60 ml der Übergangsmetall-Komplexsalz-Lösung abdekantiert und das getränkte Zinkoxid bei 130°C getrocknet. Die Konzentration an Übergangsmetall (Os) im festen Zinkoxid betrug 0,1 Gew.-%.

## Beispiel 6

Katalysatorherstellung; Übergangsmetall: Osmium.

0,84 g Ammoniumhexachloroosmat(IV) wurden in 120 ml Wasser gelöst und 180 g Zinkoxid-Preßlinge 3 h lang darin aufbewahrt, bis sie vollständig mit der Übergangsmetall-Komplexsalz-Lösung getränkt waren. Anschließend wurden 60 ml überschüssiger Metallkomplexsalz-Lösung abdekantiert und das mit Übergangsmetallsalz beaufschlagte Zinkoxid bei 130°C getrocknet. Die Konzentration an Übergangsmetall (Os) in der festen Zinkoxidbasis betrug 0,1 Gew.-%.

Hydrierung

Der in den nachfolgenden Beispielen zur Hydrierung eingesetzte Laurinsäuremethylester war ein aus Kokosöl gewonnenes Destillat (in Gewichtsprozent: gesättigt: 0 - 2 $C_{10}$; 98 - 100 $C_{12}$; 0 - 2 $C_{14}$; Säurezahl maximal 0,5; Verseifungszahl 260 - 263; Jodzahl maximal 0,2). Die Hydrierung wurde in einer üblichen, kontinuierlich arbeitenden Hydrierapparatur (HDL) durchgeführt, die aus einem 0,2 1-Reaktor ohne Einbauten oder Auskleidung, Wärmeaustauschern, Flüssigkeitspreßpumpen und Abscheidern bestand.

Für alle Versuche wurde eine Wasserstoffatmosphäre von 250 bar Druck und eine Wasserstoffmenge von 0,4 $Nm^3/h$ = 1,6 Druckliter/h verwendet.

100 ml des genannten $C_{12}$-Fettsäuremethylesters pro Stunde wurden mit einer Preßpumpe in die Hydrierapparatur gepumpt. Am zuführenden T-Stück wurde Wasserstoff unter einem Druck von 250 bar zugepreßt. Laurinsäuremethylester und Wasserstoff wurden im Vorwärmer erhitzt und über den elektrisch beheizten Festbettreaktor geleitet, der mit den wie in den Beispielen 1 bis 6 beschrieben hergestellten und anschließend 20 h bei 240°C mit molekularem Wasserstoff aktivierten festen, stückigen Katalysatoren gefüllt war. Der entstandene Laurylalkohol sowie das bei der Reaktion entstandene Methanol wurden im Kühler kondensiert und über einen Abscheider voneinander getrennt. Der Wasserstoffdruck wurde ständig bei 250 bar gehalten.

Beispiel 7

Hydrierung von Laurinsäuremethylester in der oben beschriebenen Hydrierapparatur.

Die Ergebnisse der Hydrierung mit dem Katalysator aus Beispiel 1 sind der nachfolgenden Tabelle 1 zu entnehmen.

In den nachstehenden Tabellen werden folgende Abkürzungen verwendet:
ME = Fettsäuremethylester
FA = Fettalkohol
VZ = Verseifungszahl
OHZ = Hydroxylzahl
JZ = Jodzahl
COZ = Carbonylzahl
US = Unverseifbares

Die Kennzahlen VZ, OHZ, JZ, COZ und US wurden jeweils nach den Verfahren bestimmt, die in DGF-Einheitsmethoden, Wissenschaftliche Verlagsgesellschaft mbH Stuttgart 1950 - 1984 beschrieben sind.

EP 0 241 760 B1

## Tabelle 1

### Hydrierung von Laurinsäuremethylester in der HDL

| Druck $H_2$ bar | $H_2$-Entspannung Nm³/h | Durchsatz $C_{12}$-ME ml/h | Temp. Reaktor °C | VZ | OHZ | JZ | COZ ppm | FA % | US % |
|---|---|---|---|---|---|---|---|---|---|
| 245 | 0,4 | 100 | 210 | 192 | 55 | 0,05 | 7 | 20,1 | 0,1 |
| 245 | 0,4 | 100 | 231 | 144 | 101 | 0,04 | 7 | 36,4 | 0,1 |
| 245 | 0,4 | 100 | 250 | 19,6 | 261 | 0,07 | 21 | 88 | 0,2 |
| 245 | 0,4 | 100 | 270 | 2,2 | 271 | 0,08 | 28 | 90,9 | 7,0 |
| 245 | 0,4 | 100 | 278 | 1,3 | 254 | 0,07 | 42 | 84,4 | 14,0 |

Beispiel 8

Hydrierung eines Gemisches von $C_{12}$-$C_{18}$-Fettsäuremethylestern.

Mit dem Katalysator des Beispiels 2 in der oben beschriebenen Hydrierapparatur wurde ein Gemisch von Methylestern von Fettsäuren der Kettenlänge $C_{12}$ - $C_{18}$ (in Gewichtsprozent: gesättigt: $C_{10}$ 0 - 3; $C_{12}$ 52 - 57; $C_{14}$ 19 - 23; $C_{16}$ 8 - 11; $C_{18}$ 2 - 4; einfach ungesättigt $C_{18}$ 7 - 10; zweifach ungesättigt $C_{18}$ 0 - 1; Säurezahl maximal 0,5; Verseifungszahl 235 - 245, Jodzahl 9 - 13) hydriert. Das Ergebnis ist der nachfolgenden Tabelle 2 zu entnehmen.

## Tabelle 2

### Hydrierung von $C_{12-18}$-Fettsäuremethylester in der HDL

| Druck $H_2$ bar | $H_2$-Ent-spannung Nm³/h | Durchsatz $C_{12}$-ME ml/h | Temp. Reaktor °C | VZ | OHZ | JZ | COZ ppm | FA % | US % |
|---|---|---|---|---|---|---|---|---|---|
| 250 | 0,4 | 100 | 210 | 224 | 7,3 | 10,9 | 539 | 2,7 | 0,07 |
| 250 | 0,4 | 100 | 230 | 189 | 34,1 | 10,5 | 133 | 12,6 | 0,20 |
| 250 | 0,4 | 100 | 251 | 72,7 | 160 | 7,7 | 108 | 59,2 | 0,43 |
| 250 | 0,4 | 100 | 262 | 63,7 | 172 | 11,5 | 79 | 63,7 | 0,45 |
| 250 | 0,4 | 100 | 270 | 27,1 | 227 | 5,3 | 78 | 84,1 | 0,99 |
| 250 | 0,4 | 100 | 278 | 15,0 | 253 | 1,1 | 77 | 93,7 | 3,4 |
| 250 | 0,4 | 100 | 290 | 12,0 | 247 | 2,1 | 77 | 91,5 | 6,0 |

EP 0 241 760 B1

## Beispiel 9

Hydrierung von Laurinsäuremethylester in der oben beschriebenen Hydrierapparatur.

Mit dem Katalysator aus Beispiel 3, der 0,1 Gew.-% Platin in der festen Katalysatorbasis enthielt, wurde nach Aktivierung mit Wasserstoff Laurinsäuremethylester in der oben beschriebenen Hydrierapparatur hydriert. Das Ergebnis ist der nachfolgenden Tabelle 3 zu entnehmen.

## Tabelle 3

### Hydrierung von Laurinsäuremethylester in der HDL

| Druck $H_2$ bar | $H_2$-Ent-spannung Nm³/h | Durchsatz $C_{12}$-ME ml/h | Temp. Reaktor °C | VZ | OHZ | JZ | COZ ppm | FA % | US % |
|---|---|---|---|---|---|---|---|---|---|
| 245 | 0,4 | 100 | 213 | 230 | 16 | 0,06 | 0 | 5,7 | 0,1 |
| 245 | 0,4 | 100 | 230 | 197 | 42 | 0,08 | 7 | 15,3 | 0 |
| 245 | 0,4 | 100 | 251 | 128 | 109 | 0,11 | 7 | 34,9 | 0,2 |
| 245 | 0,4 | 100 | 260 | 79 | 168 | 0,16 | 14 | 55,2 | 0,6 |
| 245 | 0,4 | 100 | 270 | 19,5 | 259 | 0,23 | 14 | 85,5 | 1,4 |
| 245 | 0,4 | 100 | 280 | 1,9 | 282 | 0,33 | 42 | 95,2 | 2,7 |
| 245 | 0,4 | 100 | 290 | 1,1 | 273 | 0,43 | 49 | 92,5 | 5,4 |

EP 0 241 760 B1

Beispiel 10

Hydrierung von Laurinsäuremethylester in der oben beschriebenen Hydrierapparatur.

Mit dem in Beispiel 4 beschriebenen Katalysator, der 0,1 % Ruthenium in der festen Katalysatorbasis enthielt, wurde Laurinsäuremethylester in der oben beschriebenen Hydrierapparatur hydriert. Die Ergebnisse sind der nachfolgenden Tabelle 4 zu entnehmen.

# Tabelle 4

Hydrierung von Laurinsäuremethylester in der HDL

| Druck $H_2$ bar | $H_2$-Entspannung Nm³/h | Durchsatz $C_{12}$-ME ml/h | Temp. Reaktor °C | VZ | OHZ | JZ | COZ ppm | FA % | US % |
|---|---|---|---|---|---|---|---|---|---|
| 245 | 0,4 | 100 | 209 | 236 | 1,5 | 0,12 | 84 | 1 | 0 |
| 245 | 0,4 | 100 | 226 | 224 | 8,5 | 0,12 | 140 | 3,2 | 0 |
| 245 | 0,4 | 100 | 251 | 186 | 27,5 | 0,14 | 84 | 9,8 | 0,2 |
| 245 | 0,4 | 100 | 269 | 7,9 | 261 | 0,54 | 105 | 86,7 | 7,5 |
| 245 | 0,4 | 100 | 280 | 3,4 | 235 | 0,58 | 112 | 79,9 | 16,6 |
| | | | 289 | 2,8 | 188 | 0,55 | 63 | 60,1 | 36 |

EP 0 241 760 B1

Beispiel 11

Hydrierung von Laurinsäuremethylester in der oben beschriebenen Hydrierapparatur.

Mit dem gemäß Beispiel 5 hergestellten Katalysator, der 0,1 % Osmium in der festen Katalysatorbasis enthielt, wurde Laurinsäuremethylester nach dem oben beschriebenen Verfahren in der Hydrierapparatur (HDL) hydriert. Die Ergebnisse sind der nachfolgenden Tabelle 5 zu entnehmen.

## Tabelle 5

### Hydrierung von Laurinsäuremethylester in der HDL

| Druck $H_2$ bar | $H_2$-Ent-spannung $Nm^3/h$ | Durchsatz $C_{12}$-ME $ml/h$ | Temp. Reaktor °C | VZ | OHZ | JZ | COZ ppm | FA % | US % |
|---|---|---|---|---|---|---|---|---|---|
| 245 | 0,4 | 100 | 211 | 238 | 1 | 0,12 | 126 | 1 | 0,3 |
| 245 | 0,4 | 100 | 230 | 231 | 3,5 | 0,12 | 154 | 1,8 | 0 |
| 245 | 0,4 | 100 | 250 | 206 | 14 | 0,13 | 140 | 6,2 | 0,2 |
| 245 | 0,4 | 100 | 270 | 138 | 78,5 | 0,23 | 112 | 30,3 | 0,7 |
| 245 | 0,4 | 100 | 290 | 33 | 232 | 0,41 | 140 | 80,2 | 4,2 |

EP 0 241 760 B1

Beispiel 12

Hydrierung von Laurinsäuremethylester in der oben beschriebenen Hydrierapparatur.

Mit dem in Beispiel 6 beschriebenen Katalysator, der 0,1 % Osmium in der festen Katalysastorbasis enthielt, wurde Laurinsäuremethylester in dem oben beschriebenen Hydrierverfahren mit Wasserstoff hydriert. Die Ergebnisse sind der nachfolgenden Tabelle 6 zu entnehmen.

## Tabelle 6

### Hydrierung von Laurinsäuremethylester in der HDL

| Druck $H_2$ bar | $H_2$-Ent- spannung $Nm^3/h$ | Durchsatz $C_{12}$-ME ml/h | Temp. Reaktor °C | VZ | OHZ | JZ | COZ ppm | FA % | US % |
|---|---|---|---|---|---|---|---|---|---|
| 245 | 0,4 | 100 | 210 | 223 | 15 | 0,08 | 224 | 5,6 | 0,1 |
| 245 | 0,4 | 100 | 230 | 212 | 23,5 | 0,11 | 70 | 8,7 | 0 |
| 245 | 0,4 | 100 | 250 | 197 | 14,5 | 0,11 | 154 | 13,3 | 0,1 |
| 245 | 0,4 | 100 | 272 | 155 | 75,5 | 0,17 | 84 | 24,6 | 0,2 |
| 245 | 0,4 | 100 | 290 | 60 | 193 | 0,45 | 70 | 61,5 | 1,8 |

EP 0 241 760 B1

Beispiel 13

Die Hydrierung wurde in einer üblichen, kontinuierlich arbeitenden Hydrierapparatur durchgeführt, die aus einem 1 l Reaktor ohne Einbauten oder Auskleidung, Wärmetauschern, Füssigkeitspreßpumpen und Abscheidern bestand.

Es wurde eine Wasserstoffatmosphäre von 250 bar Druck und eine Wasserstoffmenge von 4 $Nm^3/h$ = 16 Druckliter/h verwendet.

400 ml Laurinsäuremethylester wurden pro Stunde mit einer Preßpumpe in die Hydrierapparatur gepumpt. Am zuführenden T-Stück wurde Wasserstoff unter einem Druck von 250 bar zugepreßt. Laurinsäuremethylester und Wasserstoff wurden im Vorwärmer erhitzt und über den elektrisch beheizten Festbettreaktor geleitet, der mit 800 ml Pd-Katalysator aus Beispiel 1 hergestellten und anschließend 24 h bei 240°C mit molekularem Wasserstoff aktivierten, festen, stückigen Katalysatoren gefüllt war. Der entstandene Laurylalkohol sowie das bei der Reaktion gebildete Methanol wurden im Kühler kondensiert und über einen Abscheider voneinander getrennt. Die Menge an Wasserstoff wurde durch Konstanthalten des Druckes bei 250 bar ständig auf gleichem Niveau gehalten.

Der Versuch lief 4 Wochen und brachte gleichbleibende Ergebnisse.

VZ <1

OHZ 298

US 1 %

FA 99 %

**Patentansprüche**

1. Feste stückige Katalysatoren auf Zinkoxidbasis für die reduzierende katalytische Hydrierung von Fettsäuremethylestern und deren Mischungen zu gesättigten Fettalkoholen und deren Mischungen im Festbett, dadurch gekennzeichnet, daß sie nach Einwirken wässriger Lösungen alkalistabiler Komplexsalze der Übergangsmetalle Ruthenium, Osmium, Rhodium, Iridium, Palladium und/oder Platin auf festes, stückiges Zinkoxid und anschließende Reduktion mit gasförmigem Wasserstoff 0,01 bis 1,0 Gew.-% eines oder mehrerer der genannten Übergangsmetalle gleichmäßig in der festen Katalysatorbasis verteilt enthalten.

2. Katalysatoren nach Anspruch 1, dadurch gekennzeichnet, daß sie nach Einwirken wässriger Lösungen von Ammoniumhexachlororuthenat(IV), Ammoniumhexachloroosmat(IV), Ammoniumhexachlororhodat(IV), Tetrammin-Palladium(II)-chlorid, Diammindinitropalladium(II), Bis(acetylacetonato)palladium(II), Tetramminpalladium(II)-tetrachloropalladat(II) (Vauquelin'sches Salz), Ammoniumtetrachloroplatinat(II), Ammoniumhexachloroplatinat(IV), cis-Diammindichloroplatin(II), trans-Diammindichloroplatin(II), cis-Diammintetrachloroplatin(IV), Diammindinitritoplatin(II), Hexabromoplatin(IV)-säurenonahydrat, Kaliumhexabromoplatinat(IV), Kaliumhexaiodoplatinat(IV), Kaliumtetrabromoplatinat(II)-dihydrat, Kaliumtetrachloroplatinat(II), Kaliumtetranitritoplatinat(II), Natriumhexachloroplatinat(IV)-hexahydrat, Natriumtetrachloroplatinat(II)-tetrahydrat, Tetraamminplatin(II)-chlorid-monohydrat, Tetraamminplatin(II)-nitrat, Kaliumhexacyanoplatinat(IV), Kaliumtetracyanoplatinat(II)-hydrat, Bis(acetylacetonato)platin(II), Dichloro(ethylendiamin)platin(II), Tetrachloro(ethylendiamin)platin(IV) auf festes stückiges Zinkoxid und anschließende Reduktion mit gasförmigem Wasserstoff, 0,01 bis 1,0 Gew.-% der Übergangsmetalle Ruthenium, Osmium, Rhodium, Iridium, Palladium und/oder Platin gleichmäßig in der festen Katalysatorbasis verteilt enthalten.

3. Katalysatoren nach Ansprüchen 1 und 2, dadurch gekennzeichnet, daß sie nach Einwirken wässriger Lösungen alkalistabiler Komplexsalze des Palladiums auf festes, stückiges Zinkoxid und anschließende Reduktion mit gasförmigen Wasserstoff 0,01 bis 1,0 Gew.-% Palladium gleichmäßig in der festen Katalysatorbasis verteilt enthalten.

4. Katalysatoren nach Ansprüchen 1 bis 3, dadurch gekennzeichnet, daß sie nach Einwirken wässriger Lösungen von Tetraminpalladium(II)chlorid, Diammindinitropalladium(II), Bis(acetylacetonato)palladium(II) oder Tetramminpalladium(II)-tetrachloropalladat(II) (Vauquelin'sches Salz) auf festes stückiges Zinkoxid und anschließende Reduktion mit gasförmigem Wasserstoff 0,01 bis 1,0 Gew.-% Palladium gleichmäßig in der festen Katalysatorbasis verteilt enthalten.

5. Katalysatoren nach Ansprüchen 1 und 2, dadurch gekennzeichnet, daß sie 0,1 Gew.-% der Übergangsmetalle Ruthenium, Osmium, Rhodium, Iridium, Palladium und/oder Platin gleichmäßig in der festen Katalysatorbasis verteilt enthalten.

6. Katalysatoren nach Ansprüchen 1 bis 5, dadurch gekennzeichnet, daß sie 0,1 Gew.-% Palladium gleichmäßig in der festen Katalysatorbasis verteilt enthalten.

7. Verfahren zur katalytischen reduzierenden Hydrierung von Fettsäurealkylestern und/oder deren Mischungen zu gesättigten Fettalkoholen und/oder deren Mischungen im Festbett in Gegenwart von Wasserstoff unter Verwendung eines festen, stückigen Katalysators auf Zinkoxidbasis, dadurch gekennzeichnet, daß man

(a) festes stückiges Zinkoxid mit einer wässrigen Lösung eines oder mehrerer alkalistabiler Komplexsalze der Übergangsmetalle Ruthenium, Osmium, Rhodium, Iridium, Palladium und/oder Platin gleichmäßig tränkt,

EP 0 241 760 B1

(b) die erhaltenen Übergangsmetalloxid-beaufschlagten Zinkoxidstücke nach Trocknung in kurzer Zeit im Wasserstoffstrom bei erhöhter Temperatur reduziert und
(c) Fettsäurealkylester und/oder deren Mischungen unter Verwendung des so erhaltenen festen Katalysators bei erhöhter Temperatur und erhöhtem Wasserstoffdruck kontinuierlich oder diskontinuierlich zu gesättigten Fettalkoholen und/oder deren Mischungen hydriert.

8. Verfahren nach Anspruch 7, dadurch gekennzeichnet, daß man die Konzentration der wässrigen Lösung alkalistabiler Komplexsalze der Übergangsmetalle Ruthenium, Osmium, Rhodium, Iridium, Palladium und/oder Platin so einstellt, daß der Gehalt an Übergangsmetall in der festen Katalysatorbasis 0,01 bis 1,0 Gew.-% beträgt.

9. Verfahren nach Ansprüchen 7 und 8, dadurch gekennzeichnet, daß man die mit Übergangsmetall-Komplexsalz beaufschlagten Zinkoxidstücke trocknet und danach über einen Zeitraum von 20 h bei 240°C im Wasserstoffstrom reduziert.

## Claims

1. Solid piece-form catalysts based on zinc oxide for the reducing catalytic hydrogenation of fatty acid methyl esters and mixtures thereof to saturated fatty alcohols and mixtures thereof in a fixed bed, characterized in that, after treatment of solid piece-form zinc oxide with aqueous solutions of alkali-stable complex salts of the transition metals ruthenium, osmium rhodium iridium palladium and/or platinum on solid piece-form zinc oxide and subsequent reduction with gaseous hydrogen, the catalysts contain 0.01 to 1.0% by weight of one or more of the transition metals mentioned uniformly distributed in the solid catalyst base.

2. Catalysts as claimed in claim 1, characterized in that, after treatment of solid piece-form zinc oxide with aqueous solutions of ammonium hexachlororuthenate(IV), ammonium hexachloroosmate(IV), ammonium hexachlororhodate(IV), tetrammine palladium(II) chloride, diamminedinitropalladium(II), bis-(acetylacetonate)palladium(II), tetrammine palladium(II) tetrachloropalladate(II) (Vauquelin, s salt), ammonium tetrachloroplatinate(II), ammonium hexachloroplatinate(IV), cis-diammine dichloroplatinum(II), transdiammine dichloroplatinum(II), cis-diammine tetrachloroplatinum(IV), diamminedinitroplatinum(II), hexabromoplatinum(IV) acid nonahydrate, potassium hexabromoplatinate(IV), potassium hexaiodoplatinate(IV), potassium tetrabromoplatinate(II) dihydrate, potassium tetrachloroplatinate(II), potassium tetranitritoplatinate(II), sodium hexachloroplatinate(IV) hexahydrate, sodium tetrachloro- platinate(II) tetrahydrate, tetrammineplatinum(II) chloride monohydrate, tetrammine platinum(II) nitrate, potassium hexacyanoplatinate(IV), potassium tetracyanoplatinate(II) hydrate, bis-(acetylacetonato)-platinum(II), dichloro(ethylenediamine)platinum(II), tetrachloro(ethylenediamine)platinum(IV) on solid piece-form zinc oxide and subsequent reduction with gaseous hydrogen, the catalysts contain 0.01 to 1.0% by weight of the transition metals ruthenium, osmium, rhodium, iridium, palladium and/or platinum uniformly distributed in the solid catalyst base.

3. Catalysts as claimed in claims 1 and 2, characterized in that, after treatment of solid piece form zinc oxide with aqueous solutions of alkali-stable complex salts of palladium and subsequent reduction with gaseous hydrogen, the catalysts contain 0.01 to 1.0% by weight palladium uniformly distributed in the solid catalyst base.

4. Catalysts as claimed in claims 1 to 3, characterized in that, after treatment of solid piece form zinc oxide with aqueous solutions of tetrammine palladium(II) chloride, diammine dinitropalladium(II), bis-(acetylacetonato)palladium(II) or tetrammine palladium(II) tetrachloropalladate(II) (Vauquelin's salt) and subsequent reduction with gaseous hydrogen, the catalysts contain 0.01 to 1.0% by weight palladium uniformly distributed in the solid catalyst base.

5. Catalysts as claimed in claims 1 and 2, characterized in that they contain 0.1% by weight of the transition metals ruthenium, osmium, rhodium, iridium, palladium and/or platinum uniformly distributed in the solid catalyst base.

6. Catalysts as claimed in claims 1 to 5, characterized in that they contain 0.1% by weight palladium uniformly distributed in the solid catalyst base.

7. A process for the reducing catalytic hydrogenation of fatty acid alkyl esters and/or mixtures thereof to saturated fatty alcohols and/or mixtures thereof in a fixed bed in the presence of hydrogen using a solid piece-form catalyst based on zinc oxide, characterized in that

(a) solid piece-form zinc oxide is uniformly impregnated with an aqueous solution of one or more alkali-stable complex salts of the transition metals ruthenium, osmium, rhodium, iridium, palladium and/or platinum,
(b) after drying, the pieces of zinc oxide impregnated with transmition metal oxides are reduced in a short time in a stream of hydrogen at elevated temperature and
(c) fatty acid alkyl esters and/or mixtures thereof are continuously or discontinuously hydrogenated to saturated fatty alcohols and/or mixtures thereof at elevated temperature and elevated hydrogen pressure using the solid catalyst thus obtained.

8. A process as claimed in claim 7, characterized in that the concentration of the aqueous solution of alkali-stable complex salts of the transition metals ruthenium, osmium, rhodium, iridium, palladium and/or

platinum is adjusted in such a way that the content of transition metal in the solid catalyst base is from 0.01 to 1.0% by weight.

9. A process as claimed in claims 7 and 8, characterized in that the pieces of zinc oxide impregnated with transition metal complex salts are dried and then reduced in a stream of hydrogen for 20 h at 240°C.

**Revendications**

1. Catalyseurs solides en morceaux à base d'oxyde de zinc destinés à l'hydrogénation catalytique réductrice d'esters méthyliques d'acides gras et de leurs mélanges, en alcools gras saturés et en leurs mélanges, dans un lit fixe, ces catalyseurs étant caractérisés en ce qu'ils contiennent, après l'action de solutions aqueuses de sels complexes stables aux alcalis des métaux de transition ruthénium, osmium, rhodium, iridium, palladium et/ou platine sur l'oxyde de zinc solide en morceaux et, après réduction ultérieure avec de l'hydrogène gazeux, 0,01 à 1,0% en poids d'un ou de plusieurs des métaux de transition mentionnés répartis uniformément dans la base du catalyseur solide.

2. Catalyseurs selon la revendication 1, caractérisés en ce que, suite à l'action de solutions aqueuses d'hexachlororuthénate (IV) d'ammonium, d'hexachloroosmiate (IV) d'ammonium, d'hexachlororhodate (IV) d'ammonium, de chlorure de tétrammine-palladium (II), de diammine-dinitropalladium (II), de bis(acétyl-acétonato)palladium (II), de tétrachloropalladate (II) de tétrammine-palladium (II) (sel de Vauquelin), de tétrachloroplatinate (II) d'ammonium, d'hexa-chloro-platinate (IV) d'ammonium, de cis-diammine-dichloro-platine (II), de transdiammine-dichloroplatine (II), de cis-diamminetétrachloroplatine (IV), de diammine-dinitritoplatine (II), d'acide hexabromoplatinique (IV) monohydraté, d'hexabromoplatinate (IV) de potassium, d'hexachloroplatinate (IV) de potassium, d'hexaiodoplatinate (IV) de potassium, de tétrabromoplatinate (II) de potassium dihydraté, de tétrachloroplatinate (II) de potassium, de tétranitritoplatinate (II) de potassium, d'hexachloroplatinate (IV) de sodium hexahydraté, de tétrachloroplatinate (II) de sodium tétrahydraté, de chlorure de tétrammine-platine (II) monohydraté, de nitrate de tétrammine-platine (II), d'hexacyanoplatinate (IV) de potassium, de tétracyanoplatinate (II) de potassium hydraté, de bis(acétylacétonato)platine (II), de dichloro(éthylène-diamine)platine (II), de tétrachloro(éthylène-diamine)platine (IV) sur de l'oxyde de zinc solide en morceaux et suite à la réaction ultérieure avec de l'hydrogène gazeux, ils contiennent 0,01 à 1,0% en poids des métaux de transition ruthénium, osmium, rhodium, iridium, palladium et/ou platine répartis uniformément dans la base du catalyseur solide.

3. Catalyseurs selon les revendications 1 et 2, caractérisés en ce que, suite à l'action de solutions aqueuses de sels complexes du palladium stables aux alcalis sur de l'oxyde de zinc solide en morceaux et, suite à la réduction avec de l'hydrogène gazeux, ils contiennent 0,01 à 1,0% en poids de palladium réparti uniformément dans la base du catalyseur solide.

4. Catalyseurs selon les revendications 1 à 3, caractérisés en ce que, suite à l'action de solutions aqueuses du chlorure de tétrammine-palladium (II), du diammine-dinitro-palladium (II), du bis(acétylacétonato)palladium (II) ou du tétrachloropalladate (II) de tétrammine-palladium (II) (sel de Vauquelin) sur de l'oxyde de zinc solide en morceaux et suite à la réaction ultérieure avec de l'hydrogène gazeux, ils contiennent 0,01 à 1,0% en poids de palladium réparti uniformément dans la base du catalyseur solide.

5. Catalyseurs selon les revendications 1 et 2, caractérisés en ce qu'ils contiennent 0,1% en poids des métaux de transition ruthénium, osmium, rhodium, iridium, palladium et/ou platine, réparti uniformément dans la base du catalyseur solide.

6. Catalyseurs selon les revendications 1 à 5, caractérisés en ce qu'ils contiennent 0,1% en poids de palladium réparti uniformément dans la base du catalyseur solide.

7. Procédé destiné à l'hydrogénation catalytique réductrice d'esters alkyliques d'acides gras et/ou de leurs mélanges en alcools gras saturés et/ou en leurs mélanges, dans un lit fixe, en présence d'hydrogène, en utilisant un catalyseur solide en morceaux à base d'oxyde de zinc, caractérisé en ce que,

(a) on imprègne, uniformément, de l'oxyde de zinc solide en morceaux d'une solution aqueuse d'un ou de plusieurs sels complexes stables aux alcalis des métaux de transition ruthénium, osmium, rhodium, iridium, palladium et/ou platine;

(b) on réduit les morceaux d'oxyde de zinc obtenus auxquels on a ainsi appliqué de l'oxyde de métal de transition, après séchage de courte durée, dans un courant d'hydrogène à température élevée; et

(c) on soumet les esters alkyliques d'acides gras et/ou leurs mélanges à une hydrogénation en utilisant le catalyseur solide ainsi obtenu, à une température élevée et sous une pression élevée d'hydrogène, en continu ou en discontinu, pour obtenir des alcools gras saturés et/ou leurs mélanges.

8. Procédé selon la revendication 7, caractérisé en ce qu'on règle la concentration de la solution aqueuse des sels complexes stables aux alcalis des métaux de transition ruthénium, osmium, rhodium, iridium, palladium et/ou platine, de telle sorte que la teneur en métal de transition au sein de la base du catalyseur solide s'élève de 0,01 à 1,0% en poids.

9. Procédé selon les revendications 7 et 8, caractérisé en ce que l'on sèche les morceaux d'oxyde de zinc auxquels on a appliqué le sel complexe de métal de transition et ensuite, on les réduit dans un courant d'hydrogène, pendant une période de 20 heures à 240°C,